(19)

Europäisches Patentamt

European Patent Office

Office européen des brevets

(11) **EP 2 223 746 A1**

(12) **EUROPÄISCHE PATENTANMELDUNG**

(43) Veröffentlichungstag:
 **01.09.2010 Patentblatt 2010/35**

(51) Int Cl.:
 **B01L 3/00** (2006.01)   **G01N 33/487** (2006.01)

(21) Anmeldenummer: **09152837.2**

(22) Anmeldetag: **13.02.2009**

(84) Benannte Vertragsstaaten:
 **AT BE BG CH CY CZ DE DK EE ES FI FR GB GR HR HU IE IS IT LI LT LU LV MC MK MT NL NO PL PT RO SE SI SK TR**
 Benannte Erstreckungsstaaten:
 **AL BA RS**

(71) Anmelder:
 • **F. Hoffmann-La Roche AG**
  **4070 Basel (CH)**
  Benannte Vertragsstaaten:
  **AT BE BG CH CY CZ DK EE ES FI FR GB GR HR HU IE IS IT LI LT LU LV MC MK MT NL NO PL PT RO SE SI SK TR**
 • **Roche Diagnostics GmbH**
  **68305 Mannheim (DE)**
  Benannte Vertragsstaaten:
  **DE**

(72) Erfinder:
 • **Harttig, Herbert, Dr.**
  **67434 Neustadt/Wstr. (DE)**

 • **Roeper, Josef, Dr.**
  **67141 Neuhofen (DE)**
 • **Fuerst, Otto, Dr.**
  **68519 Viernheim (DE)**
 • **Jaeck, Thomas**
  **68542 Heddesheim (DE)**
 • **Dagenbach, Raf**
  **68723 Schwetzingen (DE)**
 • **Braun, Jürgen**
  **75365 Calw (DE)**
 • **Mönch, Ronald**
  **68549 Ilvesheim (DE)**
 • **List, Hans**
  **64754 Hesseneck-Kailbach (DE)**
 • **Koschorreck, Beate**
  **69198 Schriesheim (DE)**

(74) Vertreter: **Pfiz, Thomas et al**
 **Patentanwälte Wolf & Lutz**
 **Hauptmannsreute 93**
 **70193 Stuttgart (DE)**

(54) **Diagnostisches Testband für Flüssigproben**

(57)   Die Erfindung betrifft ein diagnostisches Testband für Flüssigproben mit einem flexiblen Transportband (12) und einer Vielzahl von auf dem Transportband (12) in Bandlängsrichtung verteilt aufgebrachten Testfeldern (14), die eine Nachweisschicht (18) und ein die Nachweisschicht (18) überspannendes Spreitnetz für eine flächige Flüssigprobenaufnahme umfassen, wobei das Spreitnetz aus einem gitterartigen Gewebe (20) mit rechtwinklig gekreuzten Gewebefäden (28,30) gebildet ist. Um eine Gewebeaufwölbung unter Bandzug zu vermeiden, wird vorgeschlagen, dass das Gewebe (20) schräg zu dem Transportband (12) orientiert ist, so dass alle Gewebefäden (28,30) schräg zur Bandlängsrichtung verlaufen.

Fig.1

**EP 2 223 746 A1**

**Beschreibung**

Die Erfindung betrifft ein diagnostisches Testband für Flüssigproben, insbesondere Körperflüssigkeiten, mit einem auf einer Spule aufgewickelten oder aufwickelbaren flexiblen Transportband und einer Vielzahl von auf dem Transportband in Bandlängsrichtung verteilt aufgebrachten Testfeldern, die eine Nachweisschicht und ein die Nachweisschicht überspannendes Spreitnetz für eine flächige Flüssigprobenaufnahme umfassen.

Solche Bandsysteme werden vor allem für Blutzuckertests konzipiert, um gegenüber den am Markt befindlichen Teststreifensystemen die Benutzerfreundlichkeit weiter zu verbessern. So kann im Sinne einer vereinfachten Handhabung auf einem aufrollbaren Transportband eine hohe Anzahl von Testeinheiten bzw. Testfeldern kompakt bevorratet und durch den Bandtransport nach Gebrauch auch wieder entsorgt werden. Aus der EP 1 593 434 geht eine einfache Montagetechnik für ein gattungsgemäßes Testband hervor. Damit ist es möglich, eine Rolle-zu-Rolle-Verarbeitung mit hoher Fertigungsgeschwindigkeit zu realisieren. Das als Spreithilfe zur verbesserten Verteilung der Flüssigprobe eingesetzte Gewebe ist dabei prozeßbedingt nur im Bereich seiner überstehenden Längsränder durch eine Klebeschicht gehalten. Löst sich das Gewebe jedoch von der Nachweisschicht, so kann es zu einer ungleichmäßigen Benetzung mit der Blutprobe kommen, wodurch eine korrekte Auswertung erschwert wird. Weiterhin kann es auch vorkommen, dass sich das Spreitgewebe erst nach der Benetzung des Testfeldes mit Blut vom Testfeld löst. Das Blut fließt dann in den noch aufliegenden Gewebebereich und bildet in dem abstehenden Gewebebereich Luftblasen, die einen negativen Einfluss auf die Messauswertung haben.

Ausgehend hiervon liegt der Erfindung die Aufgabe zugrunde, die im Stand der Technik bekannten Erzeugnisse weiter zu entwickeln und einen auch hinsichtlich der Massenfertigung optimierte robusten Bandaufbau für eine zuverlässige Probenverarbeitung anzugeben.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen.

Einem ersten Aspekt der Erfindung liegt der Gedanke zugrunde, die Dehneigenschaften des Trägerbandes und des Spreitgewebes aufeinander abzustimmen. Dementsprechend wird erfindungsgemäß vorgeschlagen, dass das Gewebe schräg zu dem Transportband orientiert ist, so dass alle Gewebefäden schräg zur Bandlängsrichtung verlaufen. Durch diese Anordnung wird vermieden, dass nur ein Fadensystem einer Zugbeanspruchung ausgesetzt ist.

Vielmehr werden alle Gewebefäden in Abhängigkeit von ihrer Schrägstellung bei einer am Trägerband angreifenden Zugkraft beansprucht, so dass sich ein bestimmtes Verhältnis von Querkontraktion und Längsdehnung auch für das Gewebe ergibt. Dadurch kann ein unterschiedlicher Querschrumpf des Gewebes und des übrigen Testbandaufbaus weitgehend reduziert werden, so dass ein ungewolltes Abheben bzw. Aufwölben der Spreithilfe vermieden wird. Vorhandene Netzmaterialen lassen sich mit geringem Aufwand an die Eigenschaften des Trägerbandes und des Testfeldes anpassen, um die Gefahr einer ungleichmäßigen Benetzung des Testfeldes mit Testflüssigkeit und daraus folgender Verfälschung des analytischen Ergebnisses weitestgehend auszuschließen.

Vorteilhafterweise ist die Orientierung des Gewebes so bestimmt, dass unter einer gegebenen Zugbelastung der Unterschied in der Querkontraktion des Transportbands und des Gewebes minimiert wird.

Abhängig von den Materialeigenschaften ist es von Vorteil, wenn das Gewebe unter einem Kompensationswinkel im Bereich zwischen 5° und 40°, vorzugsweise 20° bis 25° schräg ausgerichtet ist, wobei der Kompensationswinkel durch den kleinsten Winkel zwischen der Bandlängsrichtung und den Gewebefäden definiert ist.

Unter Berücksichtigung der Einsatzbedingungen ist es vorteilhaft, wenn das Transportband aus einem Folienmaterial mit einer Poissonzahl von 0,3 bis 0,5, vorzugsweise etwa 0,4 besteht.

Auch im Hinblick auf eine vereinfachte Herstellung ist es günstig, wenn das Gewebe in Leinwandbindung aus Kettfäden und Schussfäden gebildet ist, und wenn die Kettfäden näher zur Bandlängsrichtung als die Schussfäden verlaufen.

Eine weitere Verbesserung sieht vor, dass die gegebenenfalls hydrophil beschichteten Gewebefäden aus einem monofilen Fadenmaterial, insbesondere aus einem Polyester wie PET bestehen.

Durch die Schrägstellung ist ein einfacher Testfeldaufbau problemlos realisierbar, bei dem das Spreitnetz breiter als die Nachweisschicht ist und im Bereich seiner überstehenden Seitenränder mit einem auf das Transportband aufgebrachten, die Nachweisschicht tragenden Trägerstreifen verklebt ist.

Ein weiterer Erfindungsaspekt liegt darin, dass das Spreitnetz durch eine Abhebesicherung zusätzlich oder alternativ zu einer seitlichen Verklebung mit dem Trägerstreifen gegen Abheben von der Nachweisschicht gesichert ist. Hierbei kann auch gewährleistet werden, dass der Abstand zwischen Spreitnetz und Nachweisschicht unter Einsatzbedingungen maximal 40 Mikrometer, vorzugsweise weniger als 20 Mikrometer beträgt, so dass die Gefahr einer ungleichmäßigen Benetzung auch aufgrund von Kapillarkräften weitgehend ausgeschlossen ist.

Eine vorteilhafte Variante sieht vor, dass das rechteckförmige Spreitnetz an seinen Stirnenden jeweils eine quer zur Bandlängsrichtung verlaufende Stoffschlussverbindung, vorzugsweise eine Laserschweißnaht als Abhebesicherung aufweist. Die La.-sch-

weißnaht kann dabei herstellungstechnisch vorteilhaft durch einen Laserschnitt unter Ablängen des Testfelds von einer Rollenware gebildet sein.

Gemäß einer weiteren vorteilhaften Ausgestaltung ist das Spreitnetz breiter als die Nachweisschicht und im Bereich seiner überstehenden Seitenränder durch Klebebandstreifen als Abhebesicherung auf dem Trägerstreifen abgestützt. Dabei können die Klebebandstreifen als Abstandshalter und die Nachweisschicht eine im Wesentlichen gleiche Dicke aufweisen, so dass das Spreitnetz plan aufliegt.

In einer weiteren vorteilhaften Ausführung ist das Spreitnetz durch einen um die Nachweisschicht umlaufenden Kleberahmen als Abhebesicherung ringsum auf dem Trägerstreifen fixiert. Zu diesem Zweck kann der Kleberahmen vorteilhafterweise durch Laserverschweißung und/oder Heißklebestellen gebildet sein.

Eine weitere vorteilhafte Ausgestaltung kann darin bestehen, dass das Spreitnetz quer zur Bandlängsrichtung gleich oder weniger breit als die Nachweisschicht ist und an seinen Längsrändern durch überlappendes Klebeband als Abhebesicherung unter Freihaltung eines zentralen Applikationsfensters fixiert ist. Hierbei ist es auch vorteilhaft, wenn der das Applikationsfenster bildende Bereich aus dem Klebeband ausgestanzt ist.

Eine ebenfalls vorteilhafte Ausführung der Abhebesicherung sieht vor, dass das Spreitnetz aus einem gitterartigen Gewebe mit einem in Bandquerrichtung stufenförmig verformbaren biegesteifen Fadensystem besteht. Hierfür ist es günstig, wenn das Gewebe mit Schussfäden aus Metall versehen ist.

Eine Abhebesicherung lässt sich auch dadurch realisieren, dass der Trägerstreifen aus einem robusten Folienmaterial besteht, dessen Scherfestigkeit mehr als 0,05 N/mm$^2$ beträgt und das eine Schälfestigkeit von mehr als 1N/mm besitzt.

Im Folgenden wird die Erfindung anhand der in der Zeichnung schematisch dargestellten Ausführungsbeispiele näher erläutert. Es zeigen:

Fig. 1 ein Testband mit einem analytischen Testfeld in einer abgeschnittenen perspektivischen Darstellung;

Fig. 2 einen Querschnitt durch das Testband nach Fig. 1 im Bereich des Testfelds;

Fig. 3 eine Draufsicht auf das Testband nach Fig. 1 im Bereich eines das Testfeld überdeckenden, schräg ausgerichteten Spreitgewebes;

Fig. 4 ein Diagramm der Poissonzahl in Abhängigkeit vom Orientierungswinkel des Spreitgewebes nach Fig. 3;

Fig. 5 bis 9 einen Testbandabschnitt mit verschiedenen Ausführungsformen einer Abhebesicherung für das Spreitgewebe;

Fig. 10 ein Schema der Testfeldfertigung unter Verwendung von Rollenware; und

Fig. 11 eine diagnostische Bandkassette mit einem darin gelagerten Testband in einer aufgebrochenen perspektivischen Darstellung.

Das in der Zeichnung dargestellte diagnostische Testband 10 zur Durchführung von Blutzuckertests umfasst ein aufwickelbares flexibles Transportband 12 und eine Vielzahl von darauf für einen sukzessiven Einmalgebrauch bevorrateten, in Bandlängsrichtung voneinander beabstandeten Testelementen bzw. Testfeldern 14, die als im Umriss rechteckförmige etikettenartige Flächengebilde einen auf das Transportband 12 aufgeklebten Trägerstreifen 16, eine darauf aufgebrachte Nachweisschicht 18 und ein die Nachweisschicht 18 überspannendes Spreitnetz 20 für eine flächige Verteilung einer Probenflüssigkeit (Blutprobe) umfassen. Die Nachweisschicht 18 spricht als Trockenchemiefilm insbesondere auf Enzymbasis auf einen Analyten (Glucose) durch Farbumschlag an, so dass eine photometrische Erfassung durch den transparenten Folienverbund 12, 16 hindurch erfolgen kann.

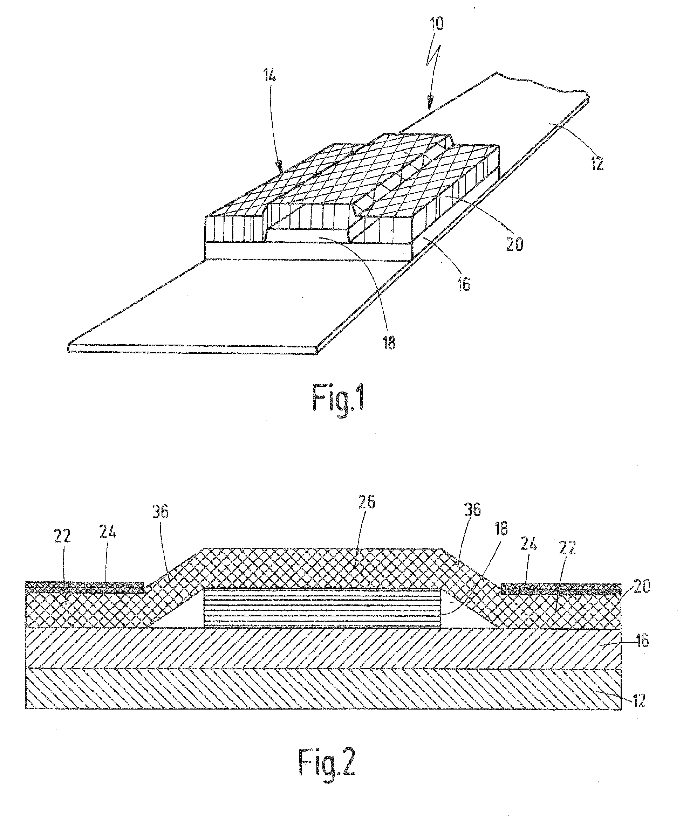

Wie aus Fig. 1 und 2 ersichtlich, ist das als Gewebe 20 ausgebildete streifenförmige Spreitnetz breiter als die Nachweisschicht 18. Die überstehenden Seitenränder 22 des Gewebes 20 sind mit der Oberseite des Trägerstreifens 16 verklebt, der seinerseits als doppelseitiges Klebebandstück auf das Transportband 12 aufgeklebt ist. An ihrer freien Außenseite sind die Seitenränder 22 des Gewebes 20 mit einer hydrophoben Beschichtung 24 versehen, so dass die Flüssigkeitsverteilung bzw. -spreitung gezielt in dem nicht verklebten Zentralbereich 26 des Gewebes 20 über der Nachweisschicht 18 erfolgt.

Die Testfelder 14 lassen sich durch Vorspulen des Transportbandes 12 an einer Applikationsstelle nacheinander zum Einsatz bringen. Aufgrund der dabei ausgeübten Zugkraft ist die flexible Bandstruktur einer Längsdehnung und Querkontraktion unterworfen, was zu einem Abheben bzw. Aufwölben des Zentralbereichs 26 des Gewebes über der Nachweisschicht 18 führen könnte. Dieser Effekt beruht darauf, dass ein in Bandlängsrichtung ausgerichtetes Gewebe durch Bandzugkräfte in seiner Länge, aber nicht in seiner Breite verändert wird, während sich bei dem Trägerband 12 durch Querkontraktion die Breite reduziert.

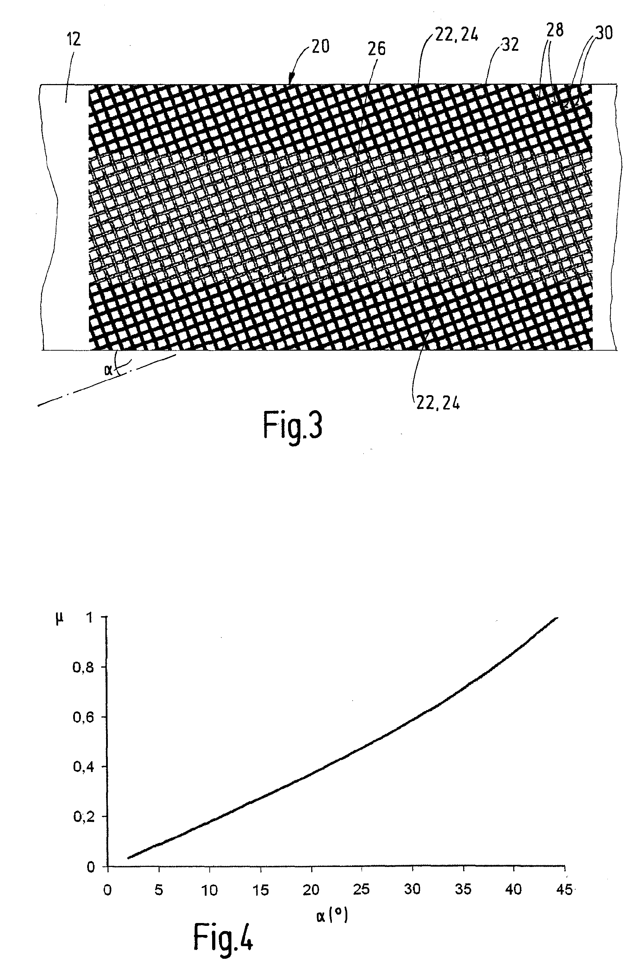

Um diesen für eine gleichmäßige Blutverteilung nachteiligen Abhebeeffekt zu vermeiden, ist das Gewebe 20 schräg zu dem Transportband 12 ausgerichtet, wie es in Fig. 3 veranschaulicht ist. Das Gewebe 20 weist rechtwinklig gekreuzte Gewebefäden 28, 30 auf, die alle schräg zur Bandlängsrichtung bzw. zu den Bandkanten 32 verlaufen.

Zweckmäßig ist das Gewebe 20 in Leinwandbindung aus Kettfäden 28 und Schussfäden 30 gebildet, die in Form von Monofilamenten aus PET bestehen. Für die Verarbeitung von Rollenware ist es günstig, wenn die langen Kettfäden 28 näher zur Bandlängsrichtung als die kurzen Schussfäden 30 verlaufen.

Die Orientierung des Gewebes 20 lässt sich durch einen Kompensationswinkel α angeben, der durch den kleinsten Winkel zwischen einer Bandkante 30 und den Gewebefäden (also in Fig. 3 den Kettfäden 28) definiert ist.

Fig. 4 zeigt die Abhängigkeit der Poissonzahl μ vom Kompensationswinkel α des Gewebes 20. Allgemein gibt die Poissonzahl die Änderung in der Querdimension d im Verhältnis zur Längsdehnung Δl eines Körpers der Länge l nach folgender Beziehung an:

$$\mu = \frac{\Delta d/d}{\Delta l/l}$$

Die Schrägausrichtung des Gewebes 20 wird so bestimmt, dass unter einer Zugbelastung der Unterschied in der Querkontraktion des Transportbands 12 und des Gewebes 20 minimiert ist. Bei einer gegebenen Poissonzahl des Trägerbandes 12 von etwa 0,4 sollte demnach der Kompensationswinkel im Bereich zwischen 20° und 25° liegen.

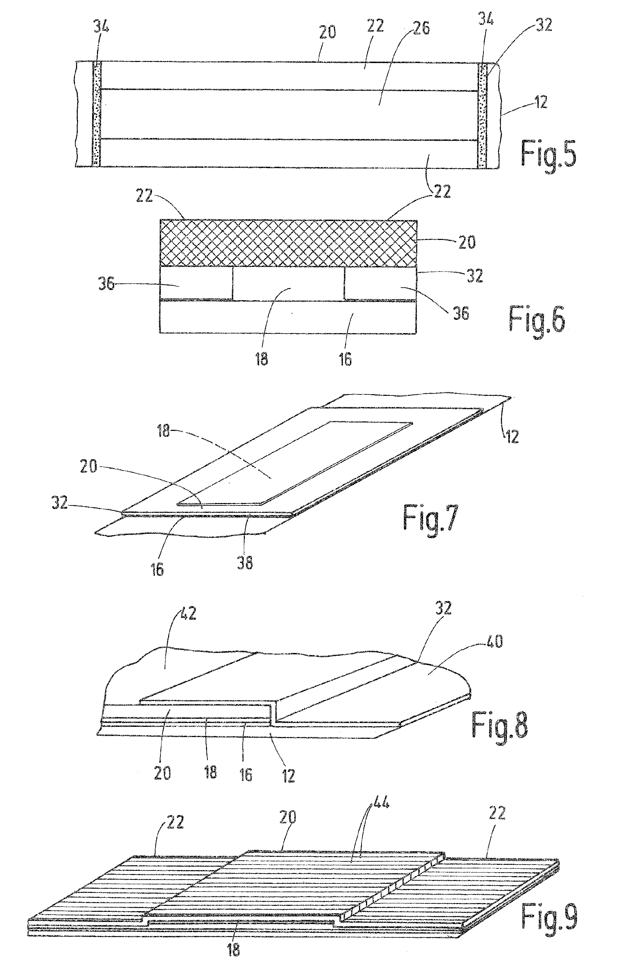

Bei den in Fig. 5 bis 9 gezeigten Ausführungsbeispielen sind gleiche oder ähnliche Teile mit gleichen Bezugszeichen entsprechend der vorstehenden Beschreibung versehen. Das Spreitnetz 20 ist dort anstelle einer Schrägstellung durch eine Abhebesicherung 32 gegen ein Aufwölben bzw. Abheben von der Nachweisschicht 18 gesichert. Diese Abhebesicherung 32 ist bei den verschiedenen Ausführungsbeispielen zusätzlich oder ergänzend zu der seitlichen Verklebung der Seitenränder 22 des Spreitnetzes 20 mit dem Trägerstreifen 16 vorgesehen. Die Ausgestaltung des Spreitnetzes 20 ist hierbei nicht nur auf Gewebe beschränkt, sondern kann auch andere Flachgebilde als Flüssigkeitsverteiler, beispielsweise eine porenhaltige Membrane umfassen. In jedem Fall wird die Abhebesicherung 32 so ausgelegt, dass der Abstand zwischen dem Zentralbereich 26 des Spreitnetzes 20 und der Nachweisschicht 18 unter Einsatzbedingungen des Testbandes maximal 40 μm, vorzugsweise weniger als 20 μm beträgt.

Bei der in Fig. 5 gezeigten Ausführungsform ist das rechteckförmige Spreitnetz 20 an seinen in Bandlängsrichtung weisenden Stirnenden jeweils durch eine quer durchgehende Laserschweißnaht 34 gesichert. Diese Fixierung kann durch eine Verschmelzung des Netzmaterials beim Laserschneiden der Testfelder 14 erfolgen, wie es nachstehend näher beschrieben ist.

Eine weitere Verbesserung der Robustheit des Testbands 10 ergibt sich dadurch, dass die in Fig. 2 erkennbare Schräge 36 im Querschnittsverlauf des Spreitnetzes 20 vermieden wird. Dies lässt sich gemäß Fig. 6 dadurch realisieren, dass das Spreitnetz 20 breiter als die Nachweisschicht 18 ist und im Bereich seiner überstehenden Seitenränder 22 durch als Abhebesicherung 32 wirkende Klebebandstreifen 36 auf dem Trägerstreifen 16 seitlich abgestützt ist. Die Klebebandstreifen 36 sollten dabei eine im Wesentlichen gleiche Dicke wie die Nachweisschicht 18 aufweisen, so dass das Spreitnetz 20 plan aufliegt.

Fig. 7 zeigt ein Ausführungsbeispiel, bei dem das Spreitnetz 20 auch stirnseitig über die Nachweisschicht 18 übersteht und an seiner Unterseite durch einen umlaufenden Kleberahmen 38 als Abhebesicherung 32 ringsum auf dem Trägerstreifen 16 fixiert ist. Dies kann durch Laserverschweißung oder eine Kombination von Klebefolien und Laserschweißen erfolgen. Eine Fertigungsmöglichkeit besteht auch darin, eine Klebefolie mit Heißklebepunkten als Zwischenträger in dem Bandfertigungsprozess einzusetzen, um passgenau Klebepunkte an den kurzen und langen Seiten des Kleberahmens 38 zu übertragen.

Bei dem in Fig. 8 gezeigten Ausführungsbeispiel besitzt das Spreitnetz 20 maximal die gleiche Breite wie die Nachweisschicht 18, so dass sich das Netz im entspannten Zustand nicht wölben kann. Alle Komponenten werden dann über seitliche Klebebänder 40 als Abhebesicherung 32 fixiert. Das Klebeband 40 überlappt somit die Längsränder des Schichtaufbaus 16,18,20, wobei ein zentrales Applikationsfenster 42 zum Auftragen der Flüssigprobe freigehalten wird. Möglich ist es auch, dass der das Applikationsfenster 42 bildende Bereich aus einem quer durchgehenden Klebebandstück 40 ausgestanzt wird.

Fig. 9 veranschaulicht ein Ausführungsbeispiel, bei dem das Spreitnetz 20 mit einem in Bandquerrichtung verlaufenden biegesteifen Fadensystem 44 als Abhebesicherung 32 versehen ist. Die Querfäden sind dabei so ausgebildet, dass sie sich weitgehend ohne Luftspalt an den Rand der Nachweisschicht 18 anpressen lassen. Dies kann zweckmäßig dadurch ermöglicht werden, dass ein Gewebe 20 mit Schussfäden aus Metall verwendet wird.

Eine weitere Möglichkeit der Robustheitsverbesserung besteht dadurch, ein geeignetes Folienmaterial für den Trägerstreifen 16 zu wählen. Ein solches Material sollte eine Scherfestigkeit im Bereich von 40N/625mm² (nach DIN EN 1943) besitzen, während die Schälfestigkeit bei etwa 25N/25mm² (nach DIN EN 1939) liegen sollte. Derartiges Folienmaterial ist beispielsweise unter dem Handelsname Duplocoll VP20242 erhältlich.

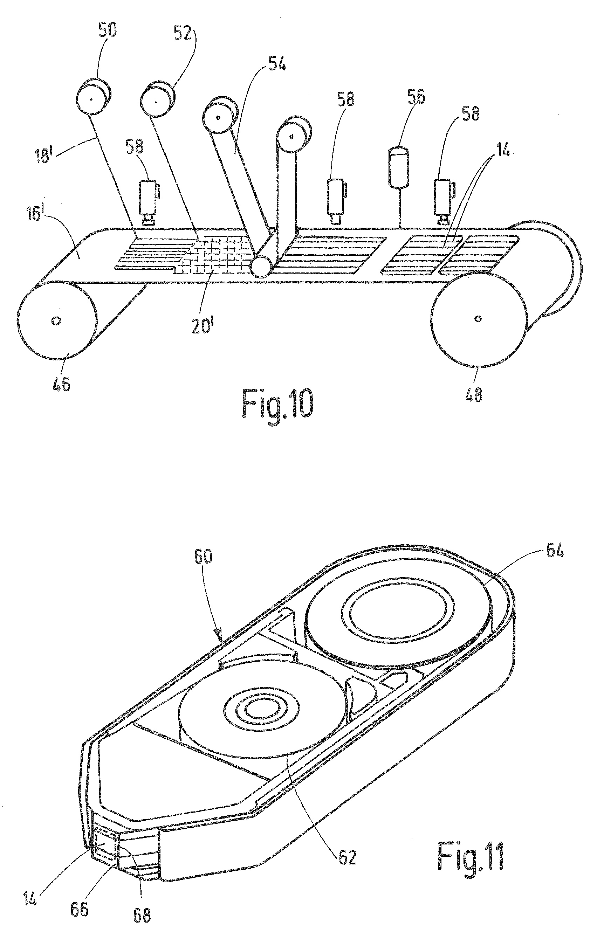

Um eine hohe Fertigungsgeschwindigkeit und -flexiblität bei der Herstellung des Testbandes 10 ermöglichen, ist ein Rolle-Zug-Rolle-Verfahren vorgesehen. Fig. 10 zeigt eine Vorstufe zur mehrspurigen Fertigung der Testfelder 14. Dabei wird ein doppelseitiges Klebeband über eine Fertigungslinie zwischen zwei Rollen 46,48 transportiert. Darauf werden von Spulen 50 mehrere parallele Nachweisfilme 18' aufgebracht. Die Anordnung wird mit Spreitgewebe 20' belegt, das von weiteren Spulen 52 abgezogen wird. Der Schichtaufbau wird anschließend mittels einer Thermotransferfolie 54 fixiert. Sodann werden mit einem Laser 56 quer verlaufende Laserschnitte eingebracht, welche die parallelen Testfelder 14 stirnseitig voneinander trennen. Der Laser 56 kann durch geeignete Wahl der Parameter nicht nur die Testfelder 14 ablängen, sondern auch die Randbereiche zur Bildung der Schweißnaht 34 (Fig. 5) untereinander verkleben. Der gesamte Prozess wird durch Kamerasysteme 58 überwacht, so dass Ausschuss weitgehend vermieden wird. Solchermaßen vorgefertigten Testfelder 14 lassen sich etikettenartig über eine Spendekante auf ein Transportband 12 übertragen und aufkleben, wobei die mehrspurig gefertigten Testbänder 10 dann längsgeteilt werden.

Das Testband 10 wird in Form einer in Fig. 11 gezeigten Bandkassette 60 als Verbrauchsmaterial in ein Handgerät eingesetzt, um es einem Patienten zu ermöglichen, eine Vielzahl von Glucosetests (beispielsweise 50 Tests) jeweils vor Ort selbst durchzuführen. Die Bandkassette 60 weist hierfür eine Abwickelspule 62 zum Abwickeln von ungebrauchtem Testband und eine Aufwickelspule 64 zum Aufwickeln von gebrauchtem Testband auf, wobei das Testband 10 über eine Applikationsspitze 66 gezogen wird, um dort die Testfelder 14 sukzessive für eine Probenaufnahme bereitzustellen. Weitere Einzelheiten der Messwerterfassung ergeben sich beispielsweise aus der EP-A 1 878 379, worauf in diesem Zusammenhang ausdrücklich Bezug genommen wird.

Beim Bandtransport wird das Testband unter einem Bandzug von ca. 4N über die Applikationsspitze 66 geführt. Um hierbei einen ungewollten Abhebeeffekt des Spreitnetzes 20 weiter zu verringern, sind die Radien der Umlenkkanten 68 geeignet abzustimmen. Durch eine Vergrößerung der Radien wird der Stress auf den Mehrkomponentenaufbau 12, 14 und damit die Aufwölbung stark verringert. Jedoch führen zu große Radien zu einer Verbreiterung der Spitze 66, und damit zu einer nachteiligen Vergrößerung der Probenauftragsfläche bzw. des benötigten Probenvolumens. In jedem Fall sollte gewährleistet sein, dass zwischen zwei Umlenkkanten 68 plan gespannt wird.

## Patentansprüche

1. Diagnostisches Testband für Flüssigproben, insbesondere Körperflüssigkeiten, mit einem auf einer Spule (62,64) aufgewickelten oder aufwickelbaren flexiblen Transportband (12) und einer Vielzahl von auf dem Transportband (12) in Bandlängsrichtung verteilt aufgebrachten Testfeldern (14), die eine Nachweisschicht (18) und ein die Nachweisschicht (18) überspannendes Spreitnetz für eine flächige Flüssigprobenaufnahme umfassen, wobei das Spreitnetz aus einem gitterartigen Gewebe (20) mit rechtwinklig gekreuzten Gewebefäden (28,30) gebildet ist, **dadurch gekennzeichnet, dass** das Gewebe (20) schräg zu dem Transportband (12) orientiert ist, so dass alle Gewebefäden (28,30) schräg zur Bandlängsrichtung verlaufen.

2. Testband nach Anspruch 1, **dadurch gekennzeichnet, dass** die Orientierung des Gewebes (20) so bestimmt ist, dass unter einer Zugbelastung der Unterschied in der Querkontraktion des Transportbands (12) und des Gewebes (20) minimiert ist.

3. Testband nach Anspruch 1 oder 2, **dadurch gekennzeichnet, dass** das Gewebe (20) unter einem Kompensationswinkel ($\alpha$) im Bereich zwischen 5° und 40°, vorzugsweise 20° bis 25° schräg ausgerichtet ist, wobei der Kompensationswinkel ($\alpha$) durch den kleinsten Winkel zwischen der Bandlängsrichtung und den Gewebefäden (28,30) definiert ist.

4. Testband nach einem der Ansprüche 1 bis 3, **dadurch gekennzeichnet, dass** das Transportband (12) aus einem Folienmaterial mit einer Poissonzahl von 0,3 bis 0,5, vorzugsweise etwa 0,4 besteht.

5. Testband nach einem der Ansprüche 1 bis 4, **dadurch gekennzeichnet, dass** das Gewebe (20) in Leinwandbindung aus Kettfäden (28) und Schussfäden (30) gebildet ist, und dass die Kettfäden (28) näher zur Bandlängsrichtung als die Schussfäden (30) verlaufen.

6. Testband nach einem der Ansprüche 1 bis 5, **dadurch gekennzeichnet, dass** die gegebenenfalls hydrophil beschichteten Gewebefäden (28,30) aus einem monofilen Fadenmaterial, insbesondere aus einem Polyester wie PET bestehen.

7. Testband nach einem der Ansprüche 1 bis 6, **dadurch gekennzeichnet, dass** das Spreitnetz (20) breiter als die Nachweisschicht (18) ist und im Bereich seiner überstehenden Seitenränder (22) mit einem auf das Transportband (12) aufgebrachten, die Nachweisschicht (18) tragenden Trägerstreifen (16) verklebt ist.

8. Diagnostisches Testband für Flüssigproben, insbesondere Körperflüssigkeiten, mit einem auf einer Spule (62,64) aufgewickelten oder aufwickelbaren flexiblen Transportband (12) und einer Vielzahl von auf dem Transportband (12) in Bandlängsrichtung verteilten Testfeldern (14), die einen auf das Transportband (12) aufgebrachten Trägerstreifen (16), eine darauf befindliche Nachweisschicht (18) und ein die Nachweisschicht (18) überspannendes Spreitnetz (20) für eine flächige Flüssigprobenaufnahme umfassen, **dadurch gekennzeichnet, dass** das Spreitnetz (20) durch eine Abhebesicherung (32) zusätzlich oder alternativ zu einer seitlichen Verklebung mit dem Trägerstreifen (16) gegen Abheben von der Nachweisschicht (18) gesichert ist.

9. Testband nach Anspruch 8, **dadurch gekennzeichnet, dass** die Abhebesicherung (32) so ausgelegt ist, dass der Abstand zwischen Spreitnetz (20) und Nachweisschicht (18) unter Einsatzbedingungen maximal 40 Mikrometer, vorzugsweise weniger als 20 Mikrometer beträgt.

10. Testband nach Anspruch 8 oder 9, **dadurch gekennzeichnet, dass** das Spreitnetz (20) rechteckförmig ist und an seinen Stirnenden jeweils eine quer zur Bandlängsrichtung verlaufende Stoffschlussverbindung (34), vorzugsweise eine Laserschweißnaht als Abhebesicherung (32) aufweist.

11. Testband nach Anspruch 8 oder 9, **dadurch gekennzeichnet, dass** das Spreitnetz (20) breiter als die Nachweisschicht (18) ist und im Bereich seiner überstehenden Seitenränder (22) durch Klebebandstreifen (36) als Abhebesicherung (32) auf dem Trägerstreifen (16) abgestützt ist.

12. Testband nach Anspruch 11, **dadurch gekennzeichnet, dass** die Klebebandstreifen (36) und die Nachweisschicht (18) eine im Wesentlichen gleiche Dicke aufweisen, so dass das Spreitnetz (20) stufenfrei abgestützt ist.

13. Testband nach Anspruch 8 oder 9, **dadurch gekennzeichnet, dass** das Spreitnetz (20) durch einen um die Nachweisschicht (18) umlaufenden Kleberahmen (38) als Abhebesicherung (32) ringsum auf dem Trägerstreifen (16) fixiert ist.

14. Testband nach Anspruch 8 oder 9, **dadurch gekennzeichnen, dass** das Spreitnetz (20) quer zur Bandlängsrichtung gleich oder weniger breit als die Nachweisschicht (18) ist und an seinen Längsrändern durch überlappendes Klebeband (40) als Abhebesicherung (32) unter Freihaltung eines zentralen Applikationsfensters (42) fixiert ist.

15. Testband nach Anspruch 8 oder 9, **dadurch gekennzeichnet, dass** das Spreitnetz (20) aus einem gitterartigen Gewebe (20) mit einem in Bandquerrichtung stufenförmig verformbaren biegesteifen Fadensystem (44) als Abhebesicherung (32) besteht.

16. Testband nach einem der Ansprüche 8 bis 15, **dadurch gekennzeichnet, dass** der Trägerstreifen (16) aus einem robusten Folienmaterial als Abhebesicherung (32) besteht, dessen Scherfestigkeit mehr als 0,05 N/mm$^2$ beträgt und das eine Schälfestigkeit von mehr als 1N/mm besitzt.

17. Bandkassette mit einem diagnostischen Testband (10) für Flüssigproben nach einem der vorhergehenden Ansprüche, wobei das Testband (10) zur Probenapplikation mit einer Bandzugkraft von mehr als IN über eine Umlenkstelle (66) geführt ist.

Fig.1

Fig.2

Fig.3

Fig.4

Fig.5

Fig.6

Fig.7

Fig.8

Fig.9

Fig.10

Fig.11

Europäisches
Patentamt

European
Patent Office

Office européen
des brevets

**EUROPÄISCHER RECHERCHENBERICHT**

**Nummer der Anmeldung**

EP 09 15 2837

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| D,X | EP 1 593 434 A (HOFFMANN LA ROCHE [CH]; ROCHE DIAGNOSTICS GMBH [DE]) 9. November 2005 (2005-11-09) * Absatz [0022] * * Absatz [0024] - Absatz [0025]; Abbildungen 2,5a-7 * ----- | 1-3, 7-15,17 | INV. B01L3/00 G01N33/487 |
| X | EP 1 522 343 A (ROCHE DIAGNOSTICS GMBH [DE]; HOFFMANN LA ROCHE [CH]) 13. April 2005 (2005-04-13) * Absatz [0043] * * Absatz [0055]; Abbildungen 3a-3d * ----- | 1-3,6-15 | |
| X | EP 1 834 696 A (HOFFMANN LA ROCHE [CH]; ROCHE DIAGNOSTICS GMBH [DE]) 19. September 2007 (2007-09-19) * Absatz [0047] - Absatz [0048] * ----- | 8,11-17 | |
| X | DE 21 18 455 B (BOEHRINGER MANNHEIM GMBH [DE]) 21. September 1972 (1972-09-21) * das ganze Dokument * ----- | 8,9, 11-16 | |
| X | EP 0 821 233 A (BOEHRINGER MANNHEIM GMBH [DE] ROCHE DIAGNOSTICS GMBH [DE]) 28. Januar 1998 (1998-01-28) * Seite 8, Zeile 23 - Seite 9, Zeile 7; Abbildungen 3-5 * ----- | 8 1-7 | RECHERCHIERTE SACHGEBIETE (IPC) B01L G01N B65C |
| A | | | |
| X | EP 0 064 710 A (BOEHRINGER MANNHEIM GMBH [DE]) 17. November 1982 (1982-11-17) * das ganze Dokument * ----- | 8 | |
| A | EP 0 209 032 A (MILES LAB [US]) 21. Januar 1987 (1987-01-21) * Seite 17, Zeile 22 - Seite 20, Zeile 9 * ----- | 8-16 | |
| A | US 5 411 858 A (MCGEEHAN JOHN K [US] ET AL) 2. Mai 1995 (1995-05-02) * das ganze Dokument * ----- | 1-16 | |

Der vorliegende Recherchenbericht wurde für alle Patentansprüche erstellt

| Recherchenort | Abschlußdatum der Recherche | Prüfer |
|---|---|---|
| München | 3. Juni 2009 | Marti, Pedro |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument

.......................................................................................

& : Mitglied der gleichen Patentfamilie, übereinstimmendes
    Dokument

EPO FORM 1503 03.82 (P04C03)

Europäisches
Patentamt
European
Patent Office
Office européen
des brevets

## GEBÜHRENPFLICHTIGE PATENTANSPRÜCHE

Die vorliegende europäische Patentanmeldung enthielt bei ihrer Einreichung Patentansprüche, für die eine Zahlung fällig war.

☐ Nur ein Teil der Anspruchsgebühren wurde innerhalb der vorgeschriebenen Frist entrichtet. Der vorliegende europäische Recherchenbericht wurde für jene Patentansprüche erstellt, für die keine Zahlung fällig war, sowie für die Patentansprüche, für die Anspruchsgebühren entrichtet wurden, nämlich Patentansprüche:

☐ Keine der Anspruchsgebühren wurde innerhalb der vorgeschriebenen Frist entrichtet. Der vorliegende europäische Recherchenbericht wurde für die Patentansprüche erstellt, für die keine Zahlung fällig war.

## MANGELNDE EINHEITLICHKEIT DER ERFINDUNG

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

```
Siehe Ergänzungsblatt B
```

☐ Alle weiteren Recherchengebühren wurden innerhalb der gesetzten Frist entrichtet. Der vorliegende europäische Recherchenbericht wurde für alle Patentansprüche erstellt.

☒ Da für alle recherchierbaren Ansprüche die Recherche ohne einen Arbeitsaufwand durchgeführt werden konnte, der eine zusätzliche Recherchengebühr gerechtfertigt hätte, hat die Recherchenabteilung nicht zur Zahlung einer solchen Gebühr aufgefordert.

☐ Nur ein Teil der weiteren Recherchengebühren wurde innerhalb der gesetzten Frist entrichtet. Der vorliegende europäische Recherchenbericht wurde für die Teile der Anmeldung erstellt, die sich auf Erfindungen beziehen, für die Recherchengebühren entrichtet worden sind, nämlich Patentansprüche:

☐ Keine der weiteren Recherchengebühren wurde innerhalb der gesetzten Frist entrichtet. Der vorliegende europäische Recherchenbericht wurde für die Teile der Anmeldung erstellt, die sich auf die zuerst in den Patentansprüchen erwähnte Erfindung beziehen, nämlich Patentansprüche:

☐ Der vorliegende ergänzende europäische Recherchenbericht wurde für die Teile der Anmeldung erstellt, die sich auf die zuerst in den Patentansprüchen erwähnte Erfindung beziehen (Regel 164 (1) EPÜ).

**Europäisches Patentamt**

**European Patent Office**

**Office européen des brevets**

**MANGELNDE EINHEITLICHKEIT DER ERFINDUNG ERGÄNZUNGSBLATT B**

**Nummer der Anmeldung**

EP 09 15 2837

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

1. Ansprüche: 1-7,17

    Ansprüche 1-7: Diagnostisches Testband für Flüssigproben dadurch gekennzeichnet, dass das Gewebe schräg zu dem Transportband orientiert ist, so dass alle Gewebefäden schräg zur Bandlängsrichtung verlaufen.
    Anspruch 17: Bandkassette mit einem diagnostischen Testband für Flüssigproben nach einem der vorhergehenden Ansprüche.
    - - -

2. Ansprüche: 8-16

    Diagnostisches Testband für Flüssigproben dadurch gekennzeichnet, dass das Spreitnetz durch eine Abhebesicherung zusätzlich oder alternativ zu einer seitlichen Verklebung mit dem Trägerstreifen gegen Abheben von der Nachweisschicht gesichert ist.
    .

    .

    Die gemeinsame Idee, die die unabhängigen Ansprüche 1 und 8 miteinander verbindet, ist durch die Merkmale im Oberbegriff beider Ansprüche definiert. Da diese gemeinsame Idee aber nicht neu (siehe Recherchenbericht) ist, sind die verschiedenen Erfindungen nicht durch eine einzige allgemeine erfinderische Idee verbunden. Darüber hinaus lösen Anspruch 1 und Anspruch 8 verschiedene technische Aufgaben, nämlich, die Anpassung der Dehneigenschaften des Trägerbandes und des Spreitgewebes bei Zugbeanspruchung (Anspruch 1) und die Absicherung des Spreitnetzes gegen Abheben von der Nachweisschicht.
    Somit ist die Anmeldung nicht einheitlich im Sinne des Art. 82 EPÜ
    - - -

**ANHANG ZUM EUROPÄISCHEN RECHERCHENBERICHT
ÜBER DIE EUROPÄISCHE PATENTANMELDUNG NR.**

EP 09 15 2837

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

03-06-2009

| Im Recherchenbericht angeführtes Patentdokument | | Datum der Veröffentlichung | Mitglied(er) der Patentfamilie | | Datum der Veröffentlichung |
|---|---|---|---|---|---|
| EP 1593434 | A | 09-11-2005 | AT | 401955 T | 15-08-2008 |
| | | | AU | 2005201576 A1 | 24-11-2005 |
| | | | BR | PI0501678 A | 23-05-2006 |
| | | | CA | 2506358 A1 | 07-11-2005 |
| | | | CN | 1693895 A | 09-11-2005 |
| | | | DK | 1593434 T3 | 17-11-2008 |
| | | | ES | 2310779 T3 | 16-01-2009 |
| | | | JP | 2005321402 A | 17-11-2005 |
| | | | KR | 20060045958 A | 17-05-2006 |
| | | | MX | PA05004199 A | 10-11-2005 |
| | | | SG | 116672 A1 | 28-11-2005 |
| | | | US | 2006002816 A1 | 05-01-2006 |
| EP 1522343 | A | 13-04-2005 | CA | 2483686 A1 | 07-04-2005 |
| | | | DE | 10346417 A1 | 02-06-2005 |
| | | | JP | 4102796 B2 | 18-06-2008 |
| | | | JP | 2005114726 A | 28-04-2005 |
| | | | US | 2005084982 A1 | 21-04-2005 |
| EP 1834696 | A | 19-09-2007 | CA | 2579186 A1 | 14-09-2007 |
| | | | CN | 101038285 A | 19-09-2007 |
| | | | JP | 2007248461 A | 27-09-2007 |
| | | | US | 2007215582 A1 | 20-09-2007 |
| DE 2118455 | B | 21-09-1972 | AT | 313243 B | 11-02-1974 |
| | | | AU | 3963072 A | 12-07-1973 |
| | | | CA | 1001537 A1 | 14-12-1976 |
| | | | CH | 534872 A | 15-03-1973 |
| | | | CS | 156535 B2 | 24-07-1974 |
| | | | DD | 95132 A5 | 12-01-1973 |
| | | | DK | 142512 B | 10-11-1980 |
| | | | ES | 400557 A1 | 01-02-1975 |
| | | | FI | 53369 B | 30-12-1977 |
| | | | FR | 2135985 A5 | 22-12-1972 |
| | | | GB | 1349623 A | 10-04-1974 |
| | | | HU | 163491 B | 27-09-1973 |
| | | | IL | 38897 A | 31-12-1974 |
| | | | IT | 953472 B | 10-08-1973 |
| | | | JP | 53006551 B | 09-03-1978 |
| | | | NL | 7202920 A | 18-10-1972 |
| | | | PL | 77544 B1 | 30-04-1975 |
| | | | RO | 64377 A1 | 15-05-1979 |
| | | | SE | 376658 B | 02-06-1975 |
| | | | SU | 524534 A3 | 05-08-1976 |
| | | | US | 3802842 A | 09-04-1974 |
| | | | YU | 61572 A | 28-02-1981 |

EPO FORM P0461

Für nähere Einzelheiten zu diesem Anhang : siehe Amtsblatt des Europäischen Patentamts, Nr.12/82

**ANHANG ZUM EUROPÄISCHEN RECHERCHENBERICHT**
**ÜBER DIE EUROPÄISCHE PATENTANMELDUNG NR.**     EP 09 15 2837

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

03-06-2009

| Im Recherchenbericht angeführtes Patentdokument | | Datum der Veröffentlichung | Mitglied(er) der Patentfamilie | | Datum der Veröffentlichung |
|---|---|---|---|---|---|
| DE 2118455 | B | | ZA | 7201509 A | 27-12-1972 |
| EP 0821233 | A | 28-01-1998 | AT | 225039 T | 15-10-2002 |
| | | | AU | 701834 B2 | 04-02-1999 |
| | | | AU | 2866097 A | 05-02-1998 |
| | | | CA | 2210652 A1 | 23-01-1998 |
| | | | CN | 1176390 A | 18-03-1998 |
| | | | CZ | 9702276 A3 | 17-06-1998 |
| | | | DE | 19629657 A1 | 29-01-1998 |
| | | | DK | 821233 T3 | 03-02-2003 |
| | | | EE | 9700163 A | 16-02-1998 |
| | | | ES | 2184012 T3 | 01-04-2003 |
| | | | HK | 1008565 A1 | 29-10-2004 |
| | | | HR | 970400 A2 | 30-04-1998 |
| | | | HU | 9701273 A2 | 28-08-1998 |
| | | | IL | 121354 A | 26-07-2000 |
| | | | JP | 3479434 B2 | 15-12-2003 |
| | | | JP | 10078433 A | 24-03-1998 |
| | | | NO | 973382 A | 26-01-1998 |
| | | | NZ | 328375 A | 27-05-1998 |
| | | | PL | 321251 A1 | 02-02-1998 |
| | | | PT | 821233 E | 28-02-2003 |
| | | | SK | 99297 A3 | 02-12-1998 |
| | | | TW | 514728 B | 21-12-2002 |
| | | | US | 5846837 A | 08-12-1998 |
| | | | ZA | 9706466 A | 22-01-1999 |
| EP 0064710 | A | 17-11-1982 | DD | 202473 A5 | 14-09-1983 |
| | | | DE | 3118381 A1 | 25-11-1982 |
| | | | JP | 1863723 C | 08-08-1994 |
| | | | JP | 5047780 B | 19-07-1993 |
| | | | JP | 57187656 A | 18-11-1982 |
| | | | SU | 1391506 A3 | 23-04-1988 |
| | | | US | 4587099 A | 06-05-1986 |
| EP 0209032 | A | 21-01-1987 | AU | 566759 B2 | 29-10-1987 |
| | | | AU | 5925586 A | 19-03-1987 |
| | | | CA | 1267596 A1 | 10-04-1990 |
| | | | DE | 3664066 D1 | 27-07-1989 |
| | | | JP | 1936074 C | 26-05-1995 |
| | | | JP | 6068488 B | 31-08-1994 |
| | | | JP | 62022063 A | 30-01-1987 |
| | | | US | 4776904 A | 11-10-1988 |
| US 5411858 | A | 02-05-1995 | KEINE | | |

EPO FORM P0461

Für nähere Einzelheiten zu diesem Anhang : siehe Amtsblatt des Europäischen Patentamts, Nr.12/82

**ANHANG ZUM EUROPÄISCHEN RECHERCHENBERICHT
ÜBER DIE EUROPÄISCHE PATENTANMELDUNG NR.**

EP 09 15 2837

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

03-06-2009

| Im Recherchenbericht angeführtes Patentdokument | Datum der Veröffentlichung | Mitglied(er) der Patentfamilie | Datum der Veröffentlichung |
|---|---|---|---|
| | | | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1593434 A
- EP 1878379 A